# EUROPEAN PATENT SPECIFICATION

(11) **EP 2 324 820 B1**
(45) Date of publication and mention of the grant of the patent: **02.03.2016**
(21) Application number: 10190058.7
(22) Date of filing: 04.11.2010
(51) Int. Cl.: A61K 9/20, A61K 9/48, A61K 31/122

(54) **Stable fluindione composition comprising one or more antioxidant agents**
Stabile Fluindionzusammensetzung mit einem oder mehreren Antioxidationsmitteln
Composition stable de fluindione comprenant un ou plusieurs agents antioxydants

(30) Priority: 04.11.2009 IN CH26762009
(43) Date of publication of application: 25.05.2011
(73) Proprietor: Inopharm Limited, 1304 Nicosia (CY)
(72) Inventor: Chandanmal Pukhraj, Bothra, 560 099, Bangalore (IN); Raju, Nadimpally Satya Varahla, 560 099, Bangalore (IN); Berthier, Leopold, 75016, PARIS (FR); Trespeuch, Florence, 78700, CONFLANS-SAINTE-HONORINE (FR); Raghupathi, Kandarapu, Hyderabad (IN); Rao Maram, Sambasiva, 522601, Narasaraopet (IN); Srinivasan, Rajamani, 607 001, Cuddalore (IN); Rao Nitturi, Manohar, 503 102, Andhrapradesh (IN); Thamma, Narendrakumar, Andhrapradesh (IN)
(74) Representative: Regimbeau

(56) References cited:
- Narendra Kumar et el.: "Stabilization of fluindione and structural elucidation of a hitherto unknown major degradation product in the tablet dosage form", , 13 October 2009 (2009-10-13), XP002616936, Retrieved from the Internet: URL:www.scientificipca.org/paper/2009/10/1 3/200910130916300A.doc [retrieved on 2011-01-14]

## Description

The present invention relates to stable pharmaceutical composition of anticoagulant. Particularly, the present invention relates to stable pharmaceutical composition of Fluindione. More particularly, the present invention relates to stable pharmaceutical composition of Fluindione further comprising one or more antioxidant agents and one or more pharmaceutically acceptable excipients.

Fluindione is an oral Indanedione anticoagulant with actions similar to those of Warfarin ((RS)-4-hydroxy-3-(3-oxo-1-phenylbutyl)-2H-chromen-2-one). It is mainly used in the management of thromboembolic disorders. Fluindione has been marketed for more than 20 years in France by Procter & Gamble Pharmaceuticals under the trademark of Previscan® 20 mg tablets.

Fluindione, a derivative of Indanedione, is a vitamin K antagonist with a long half-life. It inhibits the production of active forms of clotting factors II, VII, IX, and X. Its half-life has been reported to be about 31 hours.

Coagulation factors II, VII, IX and X and the anticoagulant proteins C and S are synthesized mainly in the liver and are biologically inactive unless 9 to 13 of amino-terminal glutamate residues are carboxylated to form the Ca²⁺-binding γ-carboxyglutamate (Gla) residues. This reaction of the descarboxy precursor proteins requires carbon dioxide, molecular oxygen and vitamins in their reduced form, and is catalyzed by γ-glutamyl cardoxylase in the rough endoplasmic reticulum. Carboxylation is directly coupled to the oxidation of vitamin K to its corresponding epoxide.

Fluindione decreases blood coagulation by inhibiting vitamin K epoxide reductase, an enzyme that recycles oxidized vitamin K to its reduced form after it has participated in the carboxylation of several blood coagulation proteins, mainly factor II and factor VII. For this reason, Fluindione is also referred to as vitamin K antagonists.

Chemically Fluindione is 2- (4-fluorophenyl)-1,3-indanedione and its molecular formula is C₁₅H₉FO₂. Structurally Fluindione is represented bellow.

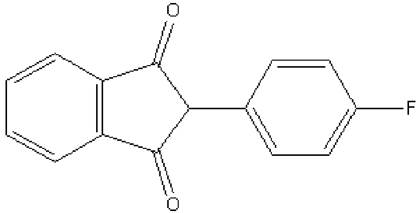

20 mg tablets contain wheat starch, anhydrous lactose, alginic acid, potato starch, stearic acid and talc as inactive ingredients.

FR 1369396 patent discloses Fluindione and its process for preparing it.

The inventors discovered that in a generic formulation, that they developed using same composition that of 20mg tablets, and in 20mg tablets, significant amounts of impurities are measured after a storage period, in particular after a storage of 3 months at 40°C/75 RH (relative humidity) conditions such as prescibed by guideline of International Conference on Harmonisation (ICH) Q1A(R2) step 4 *version.* These impurities have been identified by the inventors as being 2-(4-fluoro-phenyl)-2-hydroperoxy-indan-1,3-dione (P1) and 2-(4-fluoro-phenyl)-2-hydroxy-indan-1,3-dione (P2).

The impurity contents measured in the generic formulation and in 20mg before (initial) and after storage at 40°C/75 RH for 3 months are reported in the table 1 below:

**Table 1. Comparative Related substances data for Generic and Reference Product (Previscan®) - % by weight on the basis of the total weight of the Product**

| **Impurity name** | **Initial** | | **After storage at 40°C/75 RH for 3months** | |
|---|---|---|---|---|
| | **Previscan®** | **Generic formulation** | **Previscan®** | **Generic formulation** |
| **2-(4-fluoro-phenyl)-2-hydroperoxy-indan-1,3-dione (P1)** | 1.11% | 0.19% | 1.77% | 1.23% |
| **2-(4-fluoro-phenyl)-2-hydroxy-indan-1,3-dione (P2)** | 0.30% | 0.09% | 0.52% | 0.63% |

The impurity contents, namely the contents in 2-(4-fluoro-phenyl)-2-hydroperoxy-indan-1,3-dione (P1) and in 2-(4-fluoro-phenyl)-2-hydroxy-indan-1,3-dione (P2), measured after the 3 months storage period in both test (Generic formulation) and reference product (Previscan®) are significantly above the acceptable limits mentioned in ICH guidelines Impurities in New Drug products ICH Q3B(R2) step 4version.

As there is limited literature available for Fluindione, the reason for the presence of 2-(4-fluoro-phenyl)-2-hydroperoxy-indan-1,3-dione (P1) and 2-(4-fluoro-phenyl)-2-hydroxy-indan-1,3-dione (P2) impurities was not known. The present inventors have discovered that Fluindione is sensitive to basic conditions and oxidation conditions.

The present inventors have observed that Fluindione is get oxidised into two main oxidation products: namely one is 2-(4-fluoro-phenyl)-2-hydroperoxy-indan-1,3-dione (P1) and the other one is 2-(4-fluoro-phenyl)-2-hydroxy-indan-1,3-dione (P2). These two oxidation products were isolated and their structures were characterized by IR, ¹H NMR, ¹³ C NMR, Mass spectrometry.

From forced degradation studies the present inventors have concluded that the increased content in impurities (P1 and P2) in tablets and in the generic formulation may results from the oxidation of Fluindione.

Narendra Kumar et al. ("Stabilization of fluindione and structural elucidation of a hitherto unknown major degradation product in the tablet dosage form", 13-10-2009) disclose fluindione compositions containing antioxidants, namely citric acid, sodium citrate, ascorbic acid, sodium ascorbate, BHT and BHA. Said compositions are pharmaceutically equivalent to Previscan and better in terms of stability.

The present inventors, during their continuous efforts to develop stable Fluindione compositions, have developed a composition comprising Fluindione, one or more antioxidant agents and one or more pharmaceutically acceptable excipients thereof, which is stable in the entire shelf-life (36 months), mainly from oxidative degradation, with the consequence that the impurities content is maintained low.

The main objective of the present invention is consequently to provide stable Fluindione composition.

Specifically, the present invention provides a stable Fluindione composition comprising Fluindione, one or more antioxidant agents selected from the group consisting of sodium sulfite, sodium bisulfite, sodium metabisulfite, potassium metabisulfite, potassium bisulfite, potassium sulfite and combinations thereof, and one or more pharmaceutically acceptable excipients.

In the description of this invention, the term "stable" means that the quantitative composition does not significantly changes over the time, during the entire shelf-life of the composition, namely for at least 3 months, advantageously for at least 6 months, more advantageously for at least 12 months, even more advantageously for at least 24 months, still even more advantageously for at least 36 months. In particular, the amount in impurities does not increase during the entire shelf-life of the composition. More specifically, the content of 2-(4-fluoro-phenyl)-2-hydroperoxy-indan-1,3-dione (P1) and/or of 2-(4-fluoro-phenyl)-2-hydroxy-indan-1,3-dione (P2) does not increase during the entire shelf-life of the composition. In addition, "Stable composition" or "stable Fluindione composition" means the composition/product is stable and specifications are within the acceptable regulatory/ICH requirements during the entire shelf-life of the product in respect of assay, related substances/impurities, dissolution etc.

In an advantageous embodiment of the present invention, the stable Fluindione composition comprises less than 0.5% by weight, advantageously less than 0.4% by weight, more advantageously less than 0.3% by weight, even more advantageously less than 0.2% by weight, on the basis of the total weight of the composition, of the 2-(4-fluoro-phenyl)-2-hydroperoxy-indan-1,3-dione (P1) impurity. This content is advantageously stable during the entire shelf-life of the composition, meaning that the content in 2-(4-fluoro-phenyl)-2-hydroperoxy-indan-1,3-dione (P1) is below the above mentioned contents at the manufacturing time but also after a period of at least 3 months, advantageously after a period of at least 6 months, more advantageously after a period of at least 12 months, even more advantageously after a period of at least 24 months, still even more advantageously after a period of at least 36 months.

Structurally 2-(4-fluoro-phenyl)-2-hydroperoxy-indan-1,3-dione, referred to as P1, is depicted below:

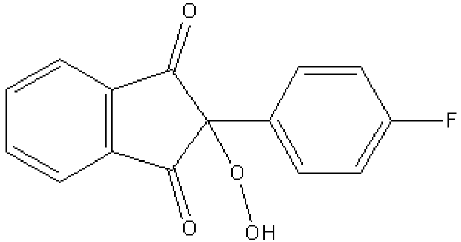

In another advantageous embodiment of the present invention, the stable Fluindione composition comprises less than 0.5% by weight, advantageously less than 0.4% by weight, more advantageously less than 0.3% by weight, even more advantageously less than 0.2% by weight, on the basis of the total weight of the composition, of 2-(4-fluoro-phenyl)-2-hydroxy-indan-1,3-dione (P2) impurity. This content is advantageously stable during the entire shelf-life of the composition, meaning that the content in 2-(4-fluoro-phenyl)-2-hydroxy-indan-1,3-dione (P2) is below the above mentioned contents at the manufacturing time but also after a period of at least 3 months, advantageously after a period of at least 6 months, more advantageously after a period of at least 12 months, even more advantageously after a period of at least 24 months, still even more advantageously after a period of at least 36 months.

Structurally 2-(4-fluoro-phenyl)-2-hydroxy-indan-1,3-dione, referred to as P2, is depicted below:

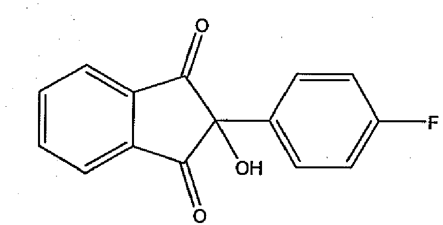

In a preferred embodiment of the invention, the stable Fluindione composition comprises, on the basis of the total weight of the composition, less than 0.5% by weight of 2-(4-fluoro-phenyl)-2-hydroperoxy-indan-1,3-dione (P1) and less than 0.5% by weight of 2-(4-fluoro-phenyl)-2-hydroxy-indan-1,3-dione (P2), at the manufacturing time but also after a period of at least 3 months, advantageously after a period of at least 6 months, more advantageously after a period of at least 12 months, even more advantageously after a period of at least 24 months, still even more advantageously after a period of at least 36 months. Advantageously, after all the above mentioned periods, the impurities content, namely the 2-(4-fluoro-phenyl)-2-hydroperoxy-indan-1,3-dione (P1) and 2-(4-fluorophenyl)-2-hydroxy-indan-1,3-dione (P2) content, is below 0.4% by weight, more advantageously below 0.3% by weight, on the basis of the total weight of the composition.

The stable Fluindione composition advantageously comprises, on the basis of the total weight of the composition, from 5% by weight to 20% by weight of Fluindione, more advantageously from 8% by weight to 15% by weight of Fluindione, still more advantageously from 9% by weight to 13% by weight of Fluindione even more advantageously from 10% by weight to 11% by weight of Fluindione.

The stable Fluindione composition advantageously comprises, on the basis of the total weight of the composition, from 0.01% to 5% by weight of antioxidant agents, more advantageously from 0.05% to 3% by weight of antioxidant agents, still more advantageously from 0.1% to 2% by weight of antioxidant agents, even more advantageously from 0.3% to 1% by weight of antioxidant agents.

The stable Fluindione composition advantageously comprises, on the basis of the total weight of the composition, from 94.99% by weight to 75% by weight of; pharmaceutically acceptable excipients, more advantageously from 91.95% by weight to 82% by weight of pharmaceutically acceptable excipients, still more advantageously from 90.9% by weight to 85% by weight of pharmaceutically acceptable excipients even more advantageously from 89.7% by weight to 88% by weight of pharmaceutically acceptable excipients.

One or more antioxidant agents that may be used according to the present invention are selected from the group consisting of sodium metabisulfite, sodium sulphite, sodium bisulphite, sodium metabisulphite, potassium metabisulphite, potassium bisulphite, potassium sulphite and combinations thereof.

It has been discovered that using these antioxidant agents (sodium metabisulfite, sodium sulphite, sodium bisulphite, sodium metabisulphite, potassium metabisulphite, potassium bisulphite, potassium sulphite and combinations thereof), both the content of 2-(4-fluoro-phenyl)-2-hydroperoxy-indan-1,3-dione (P1) and of -(4-fluorophenyl)-2-hydroxy-indan-1,3-dione (P2) is very low during the entire shelf-life of the drug.

The stable Fluindione composition advantageously comprises, on the basis of the total weight of the composition, from 0.01% to 3% by weight of the most preferred antioxidant agents, more advantageously from 0.05% to 1% by weight of the most preferred antioxidant agents, still more advantageously from 0.1% to 0.8% by weight of the most preferred antioxidant agents, even more advantageously from 0.2% to 0.5% by weight of the most preferred antioxidant agents.

One or more pharmaceutically acceptable excipients that may be used according to the present invention are selected from the group consisting of diluents, binders, disintegrants, lubricants, glidant and combinations thereof.

Suitable diluents that may be used according to the present invention are selected from the group consisting of microcrystalline cellulose, mannitol, lactose, starch, maize starch, wheat starch, potato starch, calcium hydrogen phosphate, sorbitol, sucrose, dicalcium phosphate and combinations thereof. The preferred diluent is mannitol.

Suitable binders that may be used according to the present invention are selected from the group consisting of povidone, alginic acid, starch, potato starch, wheat starch, corn starch, hydroxypropyl methylcellulose, hydroxypropyl cellulose, hydroxyethyl cellulose, gelatin and combinations thereof. The preferred binder is alginic acid.

Suitable disintegrants that may be used according to the present invention are selected from the group consisting of sodium starch glycolate, crospovidone, microcrystalline cellulose, low substituted hydroxypropyl cellulose, croscarmellose sodium, croscarmellose potassium, starch, and combinations thereof.

Suitable lubricants that may be used according to the present invention are selected from the group consisting of stearic acid, magnesium stearate, sodium stearyl fumarate, fumaric acid, calcium stearate, hydrogenated vegetable oil, stearic acid, glyceryl behenate, and combinations thereof. The preferred lubricant is stearic acid.

Suitable Glidants that may be used according to the present invention are selected from the group consisting of calcium phosphate tribasic, powdered cellulose, colloidal silicon dioxide, magnesium oxide, magnesium silicate, magnesium trisilicate, starch, talc and combinations thereof. The preferred glidant is talc.

Stable Fluindione composition according to the present invention may be in the form of immediate or modified release tablets, immediate or modified release capsules, suspension or injection.

The stable Fluindione composition of the invention is advantageously under the form of tablets. Advantageously, each tablet comprises 20 mg of Fluindione. In each tablet, the antioxidant agent content is advantageously from 0.3 to 2 mg. In each tablet, the most preferred antioxidant agent content is advantageously from 0.3 to 1 mg.

Stable Fluindione composition according to the present invention may be prepared by any suitable process, such as direct compression, wet granulation or dry granulation.

Preferably stable composition of Fluindione according to the present invention is prepared by direct compression process.

Direct compression according to the present invention advantageously comprises the following steps:
a) Optionally sifting of all materials
b) blending of all sifted materials
c) optionally lubrication
d) Compression/filled into capsules.

The invention is illustrated by the following non limiting examples:

### Comparative Example 1: Composition without antioxidant agents

**Table 2**

| **Ingredient** | **Qty mg/tablet** |
|---|---|
| Fluindione | 20.00 |
| Mannitol | 160.0 |
| Alginic acid | 10.0 |
| Talc | 3.0 |
| Stearic acid | 2.0 |
| Tablet weight | 195.0 |

### Brief description of the manufacturing process:

Fluindione and alginic acid were sifted separately through #80 mesh (177 microns) and #40mesh (420 microns) respectively and blended for 10 minutes in a blender. Purified talc and stearic acid are added to the blend. The resulting blend is then lubricated for 5 minutes and then compressed into tablets or filled into capsules.

### Example 2: Composition with Antioxidant agents:

**Table 3**

| **Ingredient** | **Qty mg/tablet** |
|---|---|
| Fluindione | 20.00 |
| Mannitol | 158.86 |
| Alginic acid | 5.00 |
| Citric acid anhydrous | 0.57 |
| Sodium citrate anhydrous | 0.57 |
| Purified Talc | 3.00 |
| Stearic acid | 2.00 |
| **Tablet weight (mg)** | **190.00** |

### Brief description of the manufacturing process:

Fluindione, alginic acid, mannitol, citric acid anhydrous, and sodium citrate anhydrous were sifted through #40 mesh (420 microns) and blended for 10 minutes in a blender, purified talc and stearic acid are added to the blend and the resulting blend is lubricated for 5 minutes and then compressed into tablets or filled into capsules.

### Example 3: Composition with Antioxidant agents

**Table 4**

| **Ingredient** | **Qty mg/tablet** |
|---|---|
| Fluindione | 20.00 |
| Mannitol | 159.43 |
| Alginic acid | 5.00 |
| Sodium metabisulfite | 0.57 |
| Purified Talc | 3.00 |
| Stearic acid | 2.00 |
| **Tablet weight (mg)** | **190.00** |

### Brief description of the manufacturing process:

Fluindione, alginic acid, mannitol, sodium metabisulfite were sifted through #40 mesh (420 microns) and blended for 10 minutes in a blender, purified talc and stearic acid are added to the blend and the resulting blend is lubricated for 5 minutes and then compressed into tablets or filled into capsules.

### Example 4: stability studies

In a first experiment, the above compressed tablets of Example 1, 2, 3 were packed in PVC/Al blisters. These blisters and (Reference product) were charged for stability at 40°C/75 RH and observed for 6 months. After 6 months, the tablets were analyzed for impurities (Related Substances) using HPLC method as follows:

**Table 5: HPLC conditions**

| HPLC Column | Phenomenex phenosphere CN 80A° 250 x 4.6 mm, 5 µm |
|---|---|
| Detector Wavelength | 235 nm |
| Flow rate | 1.0 ml / minute |
| Injection Volume | 5 µl |
| Column temperature | 30°C |
| Run time | about 45 minutes |
| Diluent | Acetonitrile: Milli-Q-Water (80:20) |

The comparative related substances data are given below:

**Table 6. Comparative Related substances data for Example 1, 2, 3 and Previscan^{®}**

| **Impurity name** | **Initial** (%by weight) | | | | **After storage at 40°C/75 RH for 6 months** (%by weight) | | | |
|---|---|---|---|---|---|---|---|---|
| | **Previscan®** | **Ex 1** | **Ex 2** | **Ex 3** | **Previscan®** | **Ex 1** | **Ex 2** | **Ex 3** |
| **Impurity P1** | 1.11 | 0.07 | 0.13 | 0.06 | 2.85 | 0.69 | 0.18 | 0.08 |
| **Impurity P2** | 0.30 | 0.06 | 0.05 | 0.09 | 0.87 | 1.47 | 0.30 | 0.10 |

In a second experiment, the above compressed tablets of Example 1, 2, 3 were packed in PVC/Al blisters. These blisters and (Reference product) were charged for stability at 25°C/60 RH and observed for 12 or 24 months. After this period, the tablets were analyzed for impurities (Related Substances) using HPLC method described above for the first experiment.

The comparative related substances data are given below:

**Table 7. Comparative Related substances data for Example 1, 2, 3 and Previscan^{®}**

| **Impurity name** | **After storage at 25°C/60 RH (% by weight)** | | | |
|---|---|---|---|---|
| | **for 24 months** | | | **for 12 months** |
| | **Previscan**® | **Ex 1** | **Ex 2** | **Ex 3** |
| **Impurity P1** | 1.66 | 0.98 | 0.23 | 0.09 |
| **Impurity P2** | 1.34 | 1.87 | 0.39 | 0.12 |

In the first and second experiments; the other tests of Dissolution and Assays are within the acceptable limits and there is no noticeable change observed after storage.

From the above data it was clearly shows that in Example 3 according to the present invention the contents in 2-(4-fluro-phenyl)-2-hydroperoxy-indan-1,3-dione (P1) and 2-(4-fluro-phenyl)-2-hydroxy-indan-1,3-dione (P2) impurities are well below 0.5% by weight, on the basis of the total weight of the composition, and thus complies as per the ICH guidelines Impurities in New Drug products.

## Claims

1. Stable Fluindione composition wherein the composition comprises Fluindione, one or more antioxidant agents selected from the group consisting of sodium sulfite, sodium bisulfite, sodium metabisulfite, potassium metabisulfite, potassium bisulfite, potassium sulfite and combinations thereof, and one or more pharmaceutically acceptable excipients.

2. Stable Fluindione composition of claim 1, wherein the content in 2-(4-fluro-phenyl)-2-hydroperoxy-indan-1,3-dione impurity (P1) is less than 0,5% by weight, on the basis of the total weight of the composition.

3. Stable Fluindione composition of claims 1 or 2, wherein the content in 2-(4-fluro-phenyl)-2-hydroxy-indan-1,3-dione impurity (P2) is less than 0,5% by weight, on the basis of the total weight of the composition.

4. Stable Fluindione composition of any one of the preceding claims, wherein one or more pharmaceutically acceptable excipients are selected from the group consisting of diluents, binders, disintegrants, lubricants, glidants and combinations thereof.

5. Stable Fluindione composition of any one of the preceding claims, wherein the composition is prepared by direct compression, wet granulation or dry granulation, most preferably by direct compression.

## Patentansprüche

1. Stabile Fluindionzusammensetzung, wobei die Zusammensetzung Fluindion, ein oder mehrere Antioxidationsmittel, die aus der Gruppe bestehend aus Natriumsulfit, Natriumhydrogensulfit, Natriumdisulfit, Kaliumdisulfit, Kaliumhydrogensulfit, Kaliumsulfit und Kombinationen davon ausgewählt sind, und ein oder mehrere pharmazeutisch akzeptable Hilfsstoffe umfasst.

2. Stabile Fluindionzusammensetzung nach Anspruch 1, wobei der Gehalt an 2-(4-Fluorphenyl)-2-hydroperoxyindan-1,3-dion-Verunreinigung (P1) weniger als 0,5 Gew.-% ausmacht, bezogen auf das Gesamtgewicht der Zusammensetzung.

3. Stabile Fluindionzusammensetzung nach Anspruch 1 oder 2, wobei der Gehalt an 2-(4-Fluorphenyl)-2-hydroxyindan-1,3-dion-Verunreinigung (P2) weniger als 0,5 Gew.-% ausmacht, bezogen auf das Gesamtgewicht der Zusammensetzung.

4. Stabile Fluindionzusammensetzung nach einem der vorhergehenden Ansprüche, wobei ein oder mehrere pharmazeutisch akzeptable Hilfsstoffe aus der Gruppe bestehend aus Verdünnungsmitteln, Bindemitteln, Sprengmitteln, Gleitmitteln, Trennmitteln und Kombinationen davon ausgewählt sind.

5. Stabile Fluindionzusammensetzung nach einem der vorhergehenden Ansprüche, wobei die Zusammensetzung durch Direktverpressung, Feuchtgranulierung oder Trockengranulierung, am meisten bevorzugt durch Direktverpressung hergestellt ist.

## Revendications

1. Composition stable de fluindione, dans laquelle la composition comprend de la fluindione, un ou plusieurs agents antioxydants sélectionnés dans le groupe consistant en le sulfite de sodium, le bisulfite de sodium, le métabisulfite de sodium, le métabisulfite de potassium, le bisulfite de potassium et le sulfite de potassium et leurs combinaisons, et un ou plusieurs excipients pharmaceutiquement acceptables.

2. Composition stable de fluindione selon la revendication 1, dans laquelle la teneur en impureté 2-(4-fluro-phényl)-2-hydroperoxy-indan-1,3-dione (P1) est inférieure à 0,5 % en poids, sur la base du poids total de la composition.

3. Composition stable de fluindione selon la revendication 1 ou 2, dans laquelle la teneur en impureté 2-(4-fluro-phényl)-2-hydroxy-indan-1,3-dione (P2) est inférieure à 0,5 % en poids, sur la base du poids total de la composition.

4. Composition stable de fluindione selon l'une quelconque des revendications précédentes, dans laquelle un ou plusieurs excipients pharmaceutiquement acceptables sont sélectionnés dans le groupe consistant en les diluants, les liants, les agents de délitement, les lubrifiants, les agents d'écoulement et leurs combinaisons.

5. Composition stable de fluindione selon l'une quelconque des revendications précédentes, dans laquelle la composition est préparée par compression directe, granulation par voie humide ou granulation par voie sèche, de manière préférée entre toutes par compression directe.
